(19) Europäisches Patentamt
European Patent Office
Office européen des brevets

(11) Numéro de publication: **0 282 798 B1**

(12) **FASCICULE DE BREVET EUROPEEN**

(45) Date de publication de fascicule du brevet: **09.09.92**

(51) Int. Cl.5: **C07C 31/137**, C07C 29/136, C07C 67/303, C07C 67/347, A61K 7/46, C11D 3/50

(21) Numéro de dépôt: **88102990.4**

(22) Date de dépôt: **29.02.88**

(54) **Alcools aliphatiques bicycliques et leur utilisation à titre d'ingrédients parfumants.**

(30) Priorité: **17.03.87 CH 1006/87**

(43) Date de publication de la demande:
**21.09.88 Bulletin 88/38**

(45) Mention de la délivrance du brevet:
**09.09.92 Bulletin 92/37**

(84) Etats contractants désignés:
**CH DE FR GB LI NL**

(56) Documents cités:
**CH-A- 529 082**
**FR-A- 2 270 232**
**GB-A- 701 911**

(73) Titulaire: **FIRMENICH SA**
**1, route des Jeunes Case Postale 239**
**CH-1211 Genève 8(CH)**

(72) Inventeur: **Giersch, Wolfgang Klaus**
**323, rue de Bernex**
**CH-1233 Bernex(CH)**
Inventeur: **Ohloff, Günther**
**13, chemin de la Chapelle**
**CH-1233 Bernex(CH)**

(74) Mandataire: **Salvadori, Giuseppe, Dr.**
**c/o Firmenich S.A. Case Postale 239**
**CH-1211 Genève 8(CH)**

**EP 0 282 798 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait au domaine de la parfumerie et plus particulièrement elle concerne l'utilisation d'alcools aliphatiques bicycliques à titre d'ingrédients parfumants pour la préparation de parfums et produits parfumés.

Parmi la variété de composés d'emploi courant en parfumerie, nombreux sont les composés destinés à conférer un caractère olfactif de type boisé aux compositions ou produits dans lesquels ils sont incorporés. Malgré le choix dont il dispose, le parfumeur se trouve souvent confronté à la nécessité de recourir à l'emploi d'ingrédients variés possédant, pour une même note de base, des nuances spécifiques voire inédites.

Nous avons maintenant découvert que les alcools bicycliques de formule

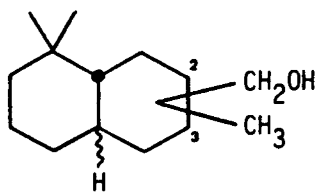

(I)

dans laquelle les substituants $CH_2OH$ et $CH_3$ sont liés de façon géminale à l'atome de carbone dans les positions 2 ou 3 du cycle hexanique et dans laquelle la ligne ondulée définit une liaison C-H de configuration cis ou trans, pouvaient servir à conférer ou améliorer la note odorante de type boisé de parfums ou produits parfumés tout en développant une note ambrée assez prononcée.

Les alcools de l'invention possèdent en effet une odeur boisée naturelle avec un côté ambré. La note boisée rappelle en particulier le bois de cèdre, sans pour autant présenter le caractère de "sciure" qui lui est propre. La note ambrée par contre évoque certains aspects présentés par des composés prisés tels l'ambre gris ou l'Ambrox (marque enregistrée de Firmenich SA). Les alcools de l'invention possèdent également une nuance douce, presque lactonique qui arrondit harmonieusement la note ambrée.

De par leurs qualités olfactives, les alcools de l'invention peuvent trouver une utilisation fort étendue tant en parfumerie alcoolique que dans des applications techniques, par exemple dans le parfumage de savons, de détergents, en poudre ou liquides, de revitalisants textiles, d'articles d'entretien ou des cosmétiques, shampoings ou crèmes de beauté, des désodorisants corporels ou d'air ambiant. Les proportions dans lesquelles les composés de l'invention servent à développer les effets odorants recherchés peuvent varier dans une gamme de valeurs assez étendue. Des concentrations préférentielles sont comprises entre 1 et 20-25%, parties en poids. De telles valeurs peuvent être inférieures lors du parfumage d'articles tels les savons, les détergents ou les cosmétiques dans lesquels les alcools (I) sont incorporés dans des proportions d'environ 0,2-1%.

Comme souvent en pareil cas, il est difficile d'estimer avec précision la quantité exacte requise pour l'emploi des alcools (I). Celle-ci dépend bien entendu de l'effet odorant spécifique recherché et de la nature des produits dans lesquels ils sont incorporés.

Les techniques de parfumage usuelles peuvent également être utilisées dans le cas de l'emploi des alcools dont il est question ici. C'est ainsi qu'ils peuvent être ajoutés soit directement au produit que l'on désire parfumer soit, plus souvent, sous forme de solution en mélange avec d'autres ingrédients parfumants habituels, des diluants ou des supports. A titre d'exemple, on peut citer à cet effet les composés naturels ou synthétiques mentionnés dans la demande de brevet européen publiée sous le n° 0096243 ou dans l'ouvrage de S. Arctander, Perfume and Flavor Chemicals, Montclair, N.J. (USA), 1969.

La formule (I) sert à définir indifféremment le perhydro-2,5,5-triméthyl-2-naphtalène-méthanol ou le perhydro-2,8,8-triméthyl-2-naphtalène-méthanol de formule

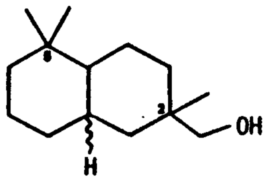 , respectivement 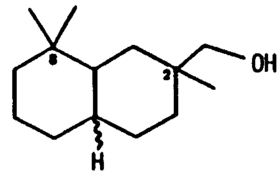

La ligne ondulée sert à définir une liaison C-H de configuration cis ou trans.

Les alcools de l'invention sont des entités chimiques nouvelles. Ils sont préparés par un procédé original à partir de myrcène selon le schéma réactionnel que voici.

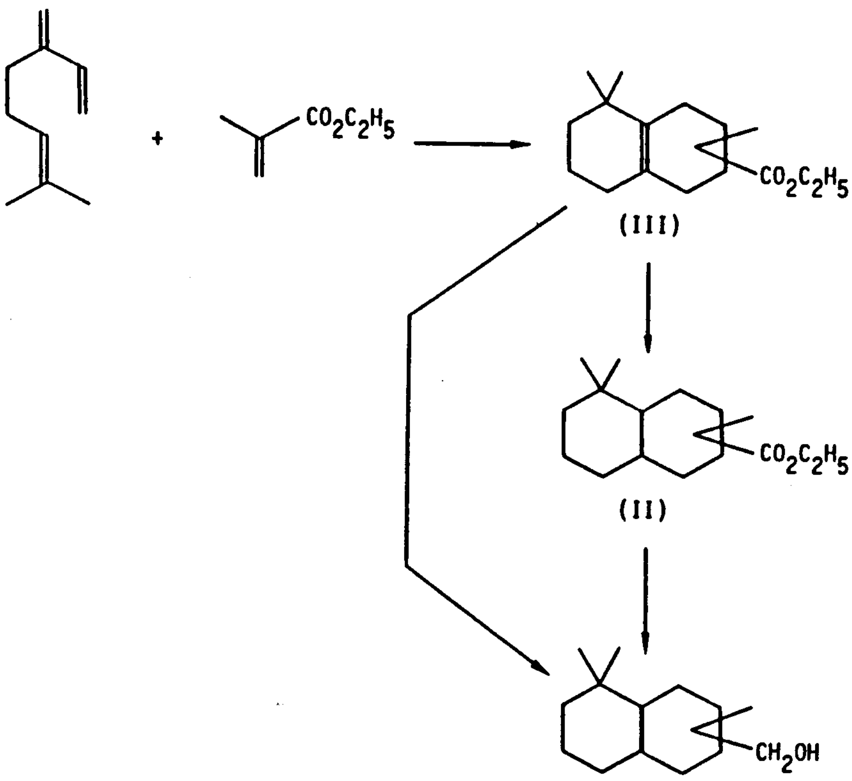

(III) (II) (I)

La première étape du procédé est caractérisée par une réaction de type Diels-Alder entre le myrcène et le méthacrylate d'éthyle, suivie par une cyclisation du produit d'addition obtenu à l'aide d'un agent de cyclisation acide. C'est ainsi qu'après traitement à température élevée (120-170° C) du mélange myrcène-méthacrylate d'éthyle dans un solvant organique inerte, on traite le mélange réactionnel avec un acide de Lewis, par exemple le trifluorure de bore sous forme d'éthérate, pour fournir l'ester (III).

La réaction s'effectue de préférence dans un milieu constitué par un solvant organique inerte choisi parmi les hydrocarbures aliphatiques ou cycloaliphatiques. A titre de solvant préférentiel, on utilise le cyclohexane. Selon un mode d'exécution particulier de l'invention, l'on opère en présence d'un inhibiteur de polymérisation et, à cet effet, on ajoute aux réactifs choisis de l'hydroquinone.

L'étape suivante consiste en une réduction de la double liaison cyclanique au moyen d'une hydrogénation catalytique. Celle-ci peut s'effectuer en présence d'un catalyseur usuel de réduction et, à cet effet, on peut employer le palladium sur charbon actif, le nickel de Raney, le nickel sur un support solide telle la silice ou l'oxyde de chrome, ou le $PtO_2$. Suivant une variante du procédé décrit, on peut obtenir les alcools (I) par réduction directe des esters (III) au moyen d'une hydrogénation catalytique en présence de catalyseurs métalliques spécifiques. Le nickel en mélange avec du chromite de cuivre peut être utilisé à cet effet. Dans ce dernier cas, la réduction a lieu de préférence dans un milieu apolaire, par exemple constitué par une hydrocarbure aromatique ou cycloaliphatique, tel le toluène ou le cyclohexane.

La dernière étape du procédé, qui consiste en la réduction de l'ester (II), peut être conduite au moyen des réactifs habituels. L'aluminohydrure de lithium convient parfaitement pour effectuer une telle réaction.

D'autres réactifs métalliques, tels le bis(2-méthoxyéthoxy)aluminohydrure de sodium [Vitride (marque enregistrée), Ethyl Corp.] ou le diéthylaluminate de sodium (OMH) peuvent être utilisés.

Grâce au procédé défini plus haut, on obtient un mélange constitué par du perhydro-2,5,5-triméthyl-2-naphtalène-méthanol et du perhydro-2,8,8-triméthyl-2-naphtalène-méthanol. Un tel mélange peut être employé directement sans séparation préalable de ses constituants pour son utilisation en parfumerie. Il peut également être soumis à une séparation au moyen des techniques habituelles, telle la chromatographie préparative en phase gazeuse. On obtient ainsi les deux alcools isomères à l'état isolé. Il est apparu à l'expérience que les propriétés olfactives des deux isomères sont très proches, néanmoins le perhydro-2,8,8-triméthyl-2-napthalène-méthanol montre des qualités plus prononcées, en particulier l'isomère ayant la structure que voici

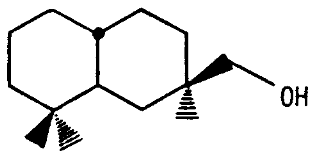

L'invention est illustrée de manière plus détaillée par les exemples qui suivent dans lesquels les températures sont indiquées en degrés centigrades et les abréviations ont le sens usuel dans l'art.

Exemple 1

a. Dans un réacteur fermé hermétiquement, on a chauffé à 160° pendant 24 h un mélange constitué par 72 g de myrcène, 66 g de méthacrylate d'éthyle, 100 g de cyclohexane et 0,2 g d'hydroquinone. Le mélange a été ensuite refroidi à température ambiante et 10 ml de trifluoroboroéthérate d'éthyle y ont été ajoutés sous azote et avec bonne agitation. La température du mélange monte sous l'effet de l'addition à environ 30° ; le tout a été ensuite laissé au repos pendant 24 h à température ambiante.
Le mélange résultant a été ensuite lavé avec 2 fractions de 50 ml chacune de NaOH à 10%, puis avec 50 ml d'eau et le cyclohexane a été enfin évaporé sous pression réduite. Par distillation sous vide du résidu, on a obtenu 86 g de 1,2,3,4,5,6,7,8-octahydro-2,5,5(2,8,8)-triméthyl-2-naphtalènecarboxylate d'éthyle ayant Eb. 90120°/0,1 mbar (rend. 65%).

b. 933 g de l'ester obtenu conformément au procédé décrit ci-dessus dans 1000 ml de méthanol ont été hydrogénés en présence de 2 g de nickel de Raney dans un autoclave à 150° et une pression de 200 bar d'hydrogène. Le mélange de réaction a été ensuite refroidi et filtré et le méthanol distillé. Le résidu ainsi obtenu a été distillé sous pression réduite pour fournir 921 g de perhydro-2,5,5(2,8,8)-triméthyl-2-naphtalènecarboxylate d'éthyle (rend. 98%) ; Eb. 81-105°/0,2 mbar.

c. 920 g de l'ester obtenu comme décrit sous lettre b. ci-dessus en solution dans 2000 ml de toluène ont été ajoutés goutte à goutte sous azote (4 h) à 1850 ml d'une solution à 25% de diéthylaluminate de sodium (OMH-1 Ethyl Corp.). Le mélange a été laissé sous agitation à température ambiante pendant 2 h supplémentaires, puis l'on a acidifié avec de l'acide sulfurique à 10%. Après lavage à l'eau et élimination du toluène par évaporation, on a obtenu un résidu qui, par distillation fractionnée, a fourni 712 g de perhydro-2,5,5(2,8,8)-triméthyl-2-naphtalène-méthanol (rend. 93%) ; Eb. : 84-90°/0,07 mbar.

Exemple 2

Une composition de base de type eau de Cologne masculine a été préparée en mélangeant les ingrédients suivants (parties en poids) :

| | |
|---|---|
| Acétate de benzyle | 200 |
| Alcool cinnamique | 50 |
| Ald. hexylcinnamique | 300 |

| | |
|---|---|
| Basilic essence | 50 |
| Essence de bergamote nat. | 3500 |
| Citral | 1000 |
| Essence de girofle | 400 |
| Essence de géranium synth. | 100 |
| Essence de lavande | 1500 |
| Méthylionone 1) | 600 |
| Dihydrojasmonate de méthyle 2) | 1000 |
| Néroli Bigarade | 150 |
| Héliopropanal | 150 |
| Vetyver Bourbon | 100 |
| | 9100 |

1) Iralia (marque enregistrée), Firmenich SA, Genève

2) Hédione (marque enregistrée), Firmenich SA Genève

En ajoutant à 91 parties de la composition de base 9 parties en poids du produit obtenu à l'Exemple 1, la composition de base acquiert un caractère plus riche avec l'apport d'une note boisée-ambrée, ce qui rend la composition plus "masculine".

Exemple 3

Une composition de base de type chypre classique a été préparée en mélangeant les ingrédients suivants (parties en poids) :

| | |
|---|---|
| Essence de bergamote nat. | 1000 |
| Essence d'orange douce | 400 |
| Rose Wardia (marque enregistrée) 1) | 800 |
| Mousse de chêne à 50% 2) | 3800 |
| Méthylionone 3) | 1000 |
| Ambre synthétique | 1000 |
| Musc cétone | 500 |
| | 8500 |

1) Firmenich SA, Genève

2) dans le dipropylèneglycol

3) Iralia (marque enregistrée), Firmenich SA, Genève

L'adjonction de 15 parties en poids du produit obtenu à l'Exemple 1 à 85 parties de la composition de base ci-dessus confère à la base une riche note boisée-ambrée. Le caractère doux de la base est modifié en même temps au profit d'une note sèche et plus lactonique.

Exemple 4

Le produit obtenu conformément à l'Exemple 1 a été ajouté à raison de 1% à une pâte de savon commercial à odeur neutre et, suivant les techniques usuelles, la pâte résultante a servi à la manufacture de barres de savon de toilette. L'article obtenu présentait une odeur agréable boisée, lactonique.

**Revendications**

**1.** Alcool aliphatique bicyclique de formule

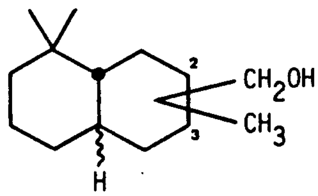

(I)

dans laquelle les substituants $CH_2OH$ et $CH_3$ sont liés de façon géminale à l'atome de carbone dans les positions 2 ou 3 du cycle hexanique et dans laquelle la ligne ondulée définit une liaison C-H de configuration cis ou trans.

**2.** Utilisation d'un alcool aliphatique bicyclique selon la revendication 1 à titre d'ingrédient parfumant pour la préparation de parfums et produits parfumés.

**3.** A titre de produit parfumé selon la revendication 2, un détergent solide ou liquide, un savon ou un adoucissant textile.

**4.** Procédé pour la préparation d'un alcool aliphatique bicyclique selon la revendication 1 caractérisé en ce qu'on

a. additionne le méthacrylate d'éthyle à du myrcène dans les conditions d'une réaction de type Diels-Alder et traite le mélange réactionnel résultant à l'aide d'un catalyseur de cyclisation acide ;

b. réduit l'ester obtenu au moyen d'une hydrogénation catalytique, et

c. réduit enfin le produit résultant au moyen d'un réactif de réduction usuel de la fonction ester.

**5.** Procédé selon la revendication 4 caractérisé en ce que les étapes b. et c. sont effectuées simultanément au moyen d'une hydrogénation catalytique en présence d'un catalyseur constitué par du Ni-$CuCr_2O_4$.

**6.** Procédé selon la revendication 4 caractérisé en ce qu'on utilise comme catalyseur de cyclisation acide un acide du type de Lewis.

**7.** Procédé selon la revendication 6 caractérisé en ce qu'on utilise comme acide de Lewis le trifluorure de bore.

**Claims**

**1.** Bicyclic aliphatic alcohol of formula

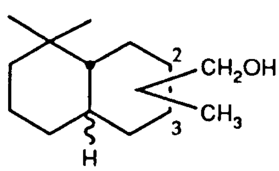

(I)

wherein substituents $CH_2OH$ and $CH_3$ are geminally bound to the carbon atom in position 2 or 3 of the

hexanic ring and wherein the wavy line stands for a C-H bond of cis or trans configuration.

**2.** Utilization of a bicyclic aliphatic alcohol according to claim 1 as a perfuming ingredient, for the preparation of perfumes and perfumed products.

**3.** As a perfumed product according to claim 2, a solid or liquid detergent, a soap or a fabric softener.

**4.** A process for the preparation of a bicyclic aliphatic alcohol according to claim 1, characterized by:
a. the addition of ethyl methacrylate to myrcene under the conditions of a Diels-Alder type reaction and the treatment of the resulting reaction mixture with an acidic cyclisation catalyst;
b. the reduction of the obtained ester by means of a catalytic hydrogenation, and
c. finally, the reduction of the resulting product by means of a current reduction reagent of the ester function.

**5.** Process according to claim 4, characterized in that steps b. and c. are carried out simultaneously by means of a catalytic hydrogenation in the presence of a catalyst consisting of Ni-$CuCr_2O_4$.

**6.** Process according to claim 4, characterized in that the acidic cyclisation catalyst is a Lewis-type acid.

**7.** Process according to claim 6, characterized in that the Lewistype acid is boron trifluoride.

**Patentansprüche**

**1.** Bicyclischer aliphatischer Alkohol der Formel

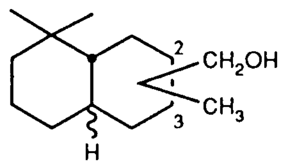

(I)

in welcher die geminalen Substituenten $CH_2OH$ und $CH_3$ an das Kohlenstoffatom 2 oder 3 des Hexanringes gebunden sind und worin die wellige Linie eine cis oder trans C-H Bindung darstellt.

**2.** Verwendung eines bicyclischen aliphatischen Alkohols gemäß Anspruch 1, als Riechstoff zur Herstellung von Parfümen und parfümierten Produkten.

**3.** Als parfümiertes Produkt gemäß Anspruch 2, ein flüssiges oder festes Reinigungsmittel, eine Seife oder ein Textilweichmacher.

**4.** Verfahren zur Herstellung eines bicyclischen aliphatischen Alkohols gemäß Anspruch 1, dadurch gekennzeichnet, daß man:
a. Methacrylsäureäthylester, unter den Diels-Alder Reaktionsbedingungen, an Myrcen addiert und die erhaltene Reaktionsmischung mittels eines sauren Cyclisierungskatalysators behandelt;
b. den erhaltenen Ester mittels einer katalischen Hydrierung reduziert, und
c. endlich, das erhaltene Produkt mittels eines für die Esterfunktion üblichen Reduktionsreagenz reduziert.

**5.** Verfahren gemäß Anspruch 4, dadurch gekennzeichnet, daß die Stufen b. und c. gleichzeitig mittels einer katalischen Hydrierung, in Gegenwart eines Ni-$CuCr_2O_4$ Katalysators, durchgeführt werden.

**6.** Verfahren gemäß Anspruch 4, dadurch gekennzeichnet, daß man als sauren Cyclisierunsgkatalysator eine Lewissäure verwendet.

**7.** Verfahren gemäß Anspruch 6, dadurch gekennzeichnet, daß man als Lewissäure Bortrifluorid verwendet.